# FASCICULE DE BREVET EUROPEEN

(11) **EP 1 568 683 B1**
(45) Date de publication et mention de la délivrance du brevet: **27.05.2009**
(21) Numéro de dépôt: 05290423.2
(22) Date de dépôt: 24.02.2005
(51) Int. Cl.: C07C 211/53, C07C 237/04, C07D 295/12, C07D 207/26, C07C 217/08, C07C 233/36, A61K 8/41, A61Q 5/10

(54) **Para-phenylenediamine secondaire n-alkylaminée, composition de teinture des fibres kératiniques contenant une tell paraphenylenediamine, procédé mettant en oeuvre cette composition et utilisations**
Sekundäre, N-alkylaminierte Para-phenylendiamine, diese enthaltende Färbezusammensetzungen für Keratinfasern, Verfahren zur Anwendung dieser Zusammensetzungen und Verwendungen
Secondary, N-alkylaminated para-phenylenediamines, compositions for dyeing keratinic fibres containing them, processes using this compositions and uses thereof

(30) Priorité: 27.02.2004 FR 0402022
(43) Date de publication de la demande: 31.08.2005
(73) Titulaire: L'ORÉAL, 75008 Paris (FR)
(72) Inventeur: Sabelle, Stéphane, 75005 Paris (FR); Genet, Alain, 93600 Aulnay sous Bois (FR)
(74) Mandataire: Dossmann, Gérard

(56) Documents cités:
- WO-A-01/64656
- WO-A-03/027102
- DE-A- 2 934 331
- FR-A- 2 315 256
- FR-A- 2 586 913
- GB-A- 1 287 343
- DATABASE CAOLD CHEMICAL ABSTRACTS SERVICE, COLUMBUS, OHIO, US; XP002295404 extrait de STN Database accession no. 63:1083A -& L. SZEKERES ET AL: ACTA PHYSIOL. ACAD. SCI. HUNG., vol. 26, no. 3, 1965, pages 287-295, XP001166995

## Description

La présente invention a pour objet une nouvelle famille de para-phénylènediamines secondaires N-alkylaminées et leur utilisation pour la teinture des fibres kératiniques, en particulier des fibres kératiniques humaines telles que les cheveux.

Il est connu de teindre les fibres kératiniques et en particulier les cheveux humains avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance à des composés colorés par un processus de condensation oxydative.

Ainsi, la demande de brevet DE 29 34 331 décrit l'utilisation de dérivés N-morpholinoalkyl diaminobenzène en teinture capillaire et GB-A-1 287 343 décrit l'utilisation de dérivés 3-aminoanilinopropionamide en teinture capillaire.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir de nouvelles compositions pour la teinture des fibres kératiniques, en particulier des fibres kératiniques humaines telles que les cheveux, capables de conduire à des colorations aux nuances variées, puissantes, esthétiques, peu sélectives et résistant bien aux diverses agressions que peuvent subir les fibres, en utilisant au moins une para-phénylènediamine secondaire N-alkylaminée.

En outre, ces compositions présentent un bon profil toxicologique.

Un premier objet de l'invention concerne une famille de para-phénylènediamines secondaires N-alkylaminées, leurs procédés de synthèse et leurs utilisations, en particulier pour la teinture des fibres kératiniques, en particulier les fibres kératiniques humaines telles que les cheveux.

L'invention a aussi pour objet une composition contenant au moins une para-phénylènediamine secondaire N-alkylaminée, les procédés de teinture mettant en oeuvre cette composition, les utilisations de la composition selon la présente invention pour la teinture des fibres kératiniques, en particulier les fibres kératiniques humaines telles que les cheveux et en particulier, les dispositifs à plusieurs compartiments ou "kits" de teinture.

La composition de la présente invention permet en particulier d'obtenir une coloration des fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Dans le cadre de la présente invention, on entend par alkyle, un radical linéaire ou ramifié en C₁-C₁₀, par exemple les radicaux linéaires ou ramifiés suivants : méthyle, éthyles, propyles, butyles, pentyles, hexyles, octyles...

Les nouvelles para-phénylènediamines secondaires N-alkylaminées selon la présente invention sont des composés de formule générale (I) : 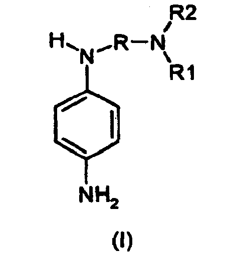 dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle,
et sous réserve que le composé de formule (I) ne soit pas le 4-[4-(pipéridinyl)butylamino]aminobenzène, ni la N-[3-(4-méthylpipérazin-1-yl)propyl]benzène 1,4-diamine, ni le 4-[2-(pipéridinyl)éthylamino]aminobenzène.

Le 4-[4-(piperidinyl)butylamino]aminobenzene est décrit dans DATABASE CAOLD CHEMICAL ABSTRACTS SERVICE, COLUMBUS, OHIO, US, XP002295404 extrait de STN Database accession n° 63:1083A ; le N-[3-(4-methylpiperazin-1-yl)propyl]benzaene-1,4-diamine est décrit dans WO 03/027102 page 77 ligne 3.22 et le 4-[2-(piperidinyl)ethylamin]aminobenzene est décrit dans WO 1/64636 page 48 lignes 20- 26 et page 49 lignes 12-17.

De manière préférée, R₁ représente un atome d'hydrogène, R₂ étant tel que défini ci-dessus ; ou les radicaux R₁ et R₂ forment ensemble avec l'azote qu'ils substituent un cycle saturé à 5 ou 6 chaînons éventuellement interrompus par un ou plusieurs atomes d'azote ou par une ou plusieurs fonctions carbonyles, et éventuellement substitué par un ou plusieurs groupements alkyles.

De manière encore préférée, R₁ et R₂ forment ensemble avec l'azote qu'ils substituent un cycle saturé à 5 ou 6 chaînons éventuellement interrompu par un ou plusieurs atomes d'azote ou par une fonction carbonyle, et éventuellement substitué par un groupement alkyle en C₁-C₃.

De manière préférée, le radical R représente un radical alkylène linéaire en C₁-C₇, de préférence C₁-C₃

De manière encore plus préférée, le radical R est non substitué ou il est interrompu par un ou plusieurs atomes choisis parmi l'azote et l'oxygène.

Les composés de formule (I) peuvent se présenter sous forme libre ou sous forme de sels, tel que les sels d'addition avec un acide de préférence choisis parmi les chlorhydrates, les bromhydrates, les sulfates, les citrates, les succinates, les tartrates, les lactates, les tosylates, les benzènesulfonates, les phosphates et les acétates.

Quelques composés de formule (I) préférés sont présentés dans le tableau qui suit :

| | | | |
|---|---|---|---|
| 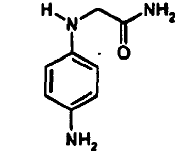 | N-[3-(4-Methylpiperazin-1-yl)-propyl]-benzene-1,4-diamine | | |
| 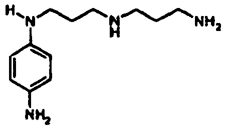 | N-[3-(3-Aminopropylamino)-propyl]-benzene-1,4-diamine | 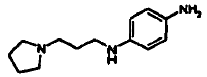 | N-(3-Pyrrolidin-1-yl-propyl)-benzene-1,4-diamine |
| 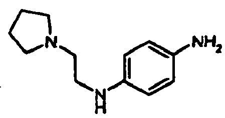 | N-(2-Pyrrolidin-1-yl-ethyl)-benzene -1,4-diamine | 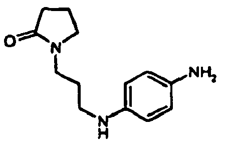 | 1-[3-(4-Aminophenylamino)-propyl]-pyrrolidin-2-one |
| 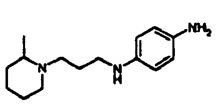 | N-[3-(2-Methylpiperidin-1-yl)-propyl]-benzene-1,4-diamine | 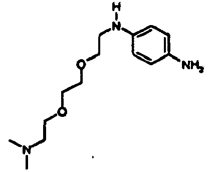 | N-{2-[2-(2-Dimethylaminoethoxy)-ethoxy]-ethyl}-benzene-1,4-diamine |

Les composés (I) selon la présente demande peuvent être préparés de façon générale suivant une méthode qui comprend les étapes suivantes :
- synthèse d'un composé 4(N-alkylaminé)nitrobenzène par substitution nucléophile de l'halogène d'un para-halogéno nitrobenzène par une diamine de formule R₁R₂NRNH₂ (R₁, R₂ et R étant tels que définis ci-dessus) en présence d'une base,
- réduction du groupement nitro du composé 4(N-alkylaminé)nitrobenzène obtenu pour obtenir le composé de formule (I) :

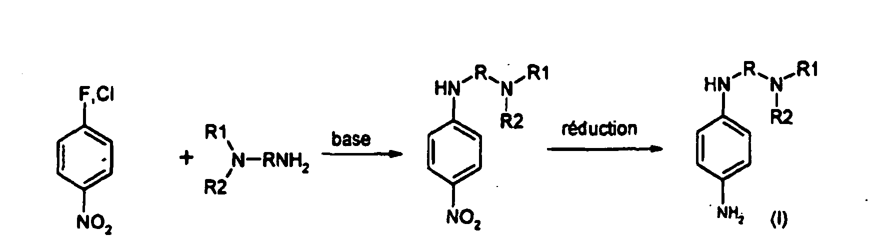

La première étape de synthèse est décrite dans les documents Synthesis 1990 (12), 1147-1148 et Synth Commun 1990, 20(22), 3537-3543.

La deuxième étape est une étape de réduction classique réalisée par exemple en effectuant une réaction d'hydrogénation par catalyse hétérogène en présence de Pd/C, Pd(II)/C, Ni/Ra... ou encore en effectuant une réaction de réduction par un métal, par exemple par du zinc, fer, étain.. (Advanced Organic Chemistry, 4th edition, 1992 J.MARCH, WILEY Interscience ; Reduction in Organic Chemistry, M.Hudlicky, 1983, Ellis Honwood series Chemical Science).

Une deuxième voie de synthèse peut être schématisée comme suit :

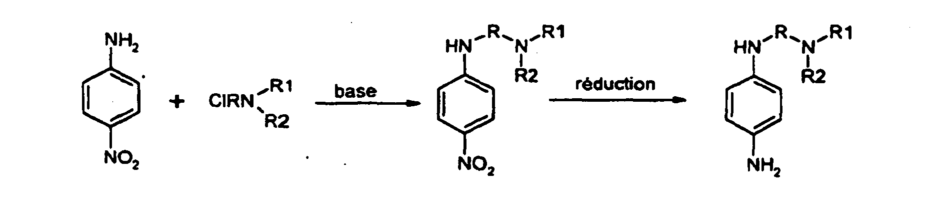

1^{ére} étape : étape réalisée en s'inspirant du J.Indian. Chem. Soc. 1990, 67, 602-603 ou du Synth. Commun.1999, 29, 1819-1933

2^{ème} étape : étape de réduction classique qui est effectuée comme exposé dans la méthode générale ci-dessus.

La présente demande concerne aussi les composés nitrés de formule (II) et les procédés de préparation des composés para-phénylènediamines secondaires de formule (I), dans lesquels on effectue une étape de réduction du composé nitré correspondant, le « composé nitré correspondant » étant le composé de formule (I) dans lequel le groupe amino en para du groupe NHRNR₁R₂ est remplacé par un groupe nitro.

Ces composés correspondent à la formule : 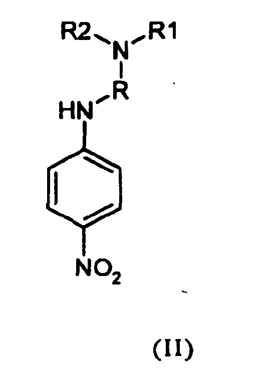 dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompus par un ou plusieurs atomes choisis parmi l'azote et l'oxygène, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle.

La présente demande concerne également les utilisations des composés de formule générale (I) selon la présente demande, et en particulier, l'utilisation des composés de formule générale (I) selon la présente demande dans laquelle les radicaux R1 et R2 représentent, indépendamment l'un de l'autre, un atome d'hydrogène, un groupement alkyle, un groupement monoalkylaminocarbonyle, un groupement dialkylaminocarbonyle, ou les radicaux R1 et R2 forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C1-C10, linéaire ou ramifié, éventuellement interrompu par un ou plusieurs atomes choisis parmi l'azote et l'oxygène ou par une ou plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C1-C10, linéaire ou ramifié, non substitué et non interrompu par un ou plusieurs atomes choisis parmi l'azote et l'oxygène ou par une ou plusieurs fonctions carbonyles, alors les radicaux R1 et R2 ne représentent pas l'un des couples suivants : deux atomes d'hydrogène, deux groupements alkyles, un atome d'hydrogène et un groupement alkyle, pour la teinture des fibres kératiniques, de préférence les fibres kératiniques humaines telles que les cheveux.

La présente demande concerne aussi une composition cosmétique pour la teinture des fibres, de préférence les fibres kératiniques telles que les cheveux, comprenant, dans un milieu approprié pour la teinture, au moins un composé de formule générale (I) dans laquelle les radicaux R1 et R2 représentent, indépendamment l'un de l'autre, un atome d'hydrogène, un groupement alkyle, un groupement monoalkylaminocarbonyle, un groupement dialkylaminocarbonyle, ou les radicaux R1 et R2 forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C1-C10, linéaire ou ramifié, éventuellement interrompu par un ou plusieurs atomes choisis parmi l'azote et l'oxygène ou par une plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle, pour la teinture des fibres kératiniques, de préférence les fibres kératiniques humaines telles que les cheveux. La présente demande concerne également une composition cosmétique pour la teinture des fibres, de préférence les fibres kératiniques telles que les cheveux, comprenant, dans un milieu approprié pour la teinture, au moins un composé de formule générale (I) et au moins un adjuvant cosmétique choisi dans le groupe formé par les agents antioxydants, les agents de pénétration, les agents séquestrants, les parfums, les tampons, les agents dispersants, les tensioactifs, les conditionneurs, les agents filmogènes, les polymères, les céramides, les agents conservateurs, les agents nacrants ou opacifiants, les vitamines ou provitamines.

La présente demande concerne également l'utilisation d'une composition cosmétique comprenant, dans un milieu approprié pour la teinture, au moins un composé de formule générale (I), dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène ou par une ou plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle, pour la teinture des fibres, de préférence les fibres kératiniques telles que les cheveux.

De préférence, la concentration du composé de formule générale (I) est comprise entre 0,0001 et 20%, de préférence entre 0,005 à 6% en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Avantageusement, la composition cosmétique comprend au moins un adjuvant cosmétique choisi dans le groupe formé par les agents antioxydants, les agents de pénétration, les agents séquestrants, les parfums, les tampons, les agents dispersants, les tensioactifs, les conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un précurseur de colorant d'oxydation additionnel différent des composés de formule (I), de manière encore plus préférée la composition selon l'invention contient au moins un coupleur.

Parmi les coupleurs d'oxydation, on peut notamment citer les méta-phénylènediamines, les métaaminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

Les bases d'oxydation additionnelles différentes des composés de formule (I) peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para--aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6 dichlorophénol, le 4-amino 6[((5'amino-2'hydroxy-3'-méthyl)phényl)méthyl]-2-méthylphénol, le bis[(5'amino-2'hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration d la ou des bases d'oxydation est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1 H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Bien entendu, l'homme de l'art veillera à choisir le ou les adjuvants, précurseurs de colorants d'oxydations additionnels, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide ortho-phosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 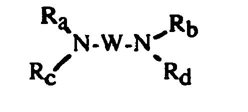 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition cosmétique selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La présente demande concerne un procédé dans lequel on applique sur les fibres kératiniques la composition selon la présente invention telle que définie précédemment pendant une durée suffisante pour développer la coloration désirée en présence d'un agent oxydant, l'agent oxydant étant appliqué avant, simultanément ou après la composition. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration : selon ce mode de réalisation particulier, on dispose d'une composition prête à l'emploi qui est un mélange d'une composition selon l'invention avec au moins un agent oxydant de préférence choisi parmi le peroxyde d'hydrogène, le peroxyde d'urée, les bromates de méta-ux alcalins, les persels, les peracides et les enzymes oxydases. Le mélange obtenu, sous la forme d'une composition prête à l'emploi, est ensuite appliqué sur les fibres kératiniques pendant une durée suffisante pour développer la coloration désirée. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de méta-ux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES DE SYTNHESE

### Exemple 1 : Synthèse du N-[3-(3-Amino-propylamino)-propyl]-benzène-1,4-diamine, tétrachlorhydrate (2)

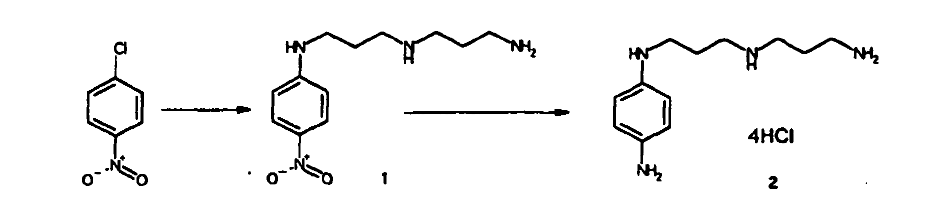

### Etape 1 : Préparation du N-(3-Amino-propyl)-N'-(4-nitro-phényl)-propane-1,3-diamine (1)

On chauffe au bain-marie bouillant 105 g (0,8 mole) de N-1-(3-amino-propyl)-propane-1,3-diamine puis on y ajoute en 1h une solution tiède de p-chloronitrobenzène (31,5g, 0,2 mole) dans 35 ml de pyridine tout en maintenant le chauffage pendant 3 heures. Lorsque la réaction est totale, le milieu est versé sur 1L d'eau glacée. On laisse cristalliser lentement à 0°C puis on essore et on lave le solide jaune formé à l'eau glacée puis on sèche à 40°C sur P₂O₅. Le produit obtenu (37,3 g) est recristallisé dans 70 ml d'acétonitrile pour donner 26,2 g de cristaux qui sont recristallisés à nouveau dans 40 ml d'acétonitrile. 19,9 g de produit monohydraté ont été isolés (Fusion = 64°C) avec les analyses élémentaires suivantes :

| | **THEORIE, H₂O** | **TROUVE** |
|---|---|---|
| **C** | 53.32 | 53.30 |
| **H** | 8.20 | 8.11 |
| **N** | 20.73 | 20.76 |
| **Cl** | 17.76 | 18.08 |

### Etape 2 : Préparation du N-[3-(3-Amino-propylamino)-propyl]-benzène-1,4-diamine, tétrachlorhydrate (2)

Le mélange de zinc en poudre (32g), de chlorure d'ammonium (1,3g), d'eau (6,8 ml) et d'éthanol 96% (65 ml) est porté au reflux au bain-marie bouillant. Le dérivé N-(3-Amino-propyl)-N'-(4-nitro-phényl)-propane-1,3-diamine (1) (16,5 g ; 6,5x10⁻² mole) est ajouté petit à petit et le chauffage est maintenu jusqu'à décoloration du milieu réactionnel. On filtre à chaud et le filtrat est récupéré sur 50 ml d'éthanol chlorhydrique 7N froid. Le zinc est lavé avec un minimum d'éthanol bouillant et le filtrat est laissé refroidi à 0°C. Les cristaux blancs formés sont essorés, lavés à l'éther éthylique et séchés sous vide à 45°C et sur P₂O₅/KOH pour donner 21,1 g de produits tétrachlorhydratés

| | **THEORIE, 4HCl** | **TROUVE** |
|---|---|---|
| **C** | 39.15 | 39.14 |
| **H** | 7.12 | 7.05 |
| **N** | 15.22 | 15.13 |
| **Cl** | 38.52 | 38.54 |

Les spectres RMN du proton et de masse sont conformes à la structure attendue du produit.

### Exemple 2: Synthèse de 2-(4-Amino-phénylamino)-acétamide, dichlorhydrate (4)

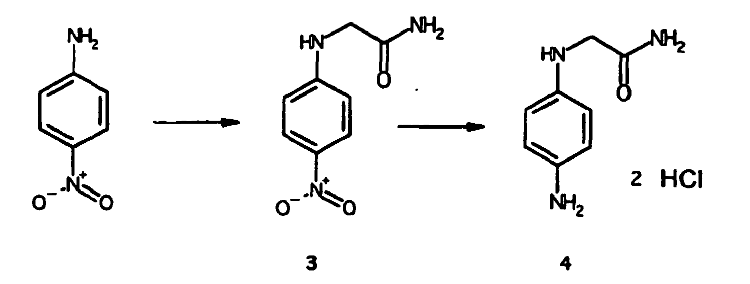

### Etape 1 : Préparation de 2-(4-Nitro-phénylamino)-acétamide (3)

Le mélange de p-nitroaniline (55,2g, 0,4 mole), de carbonate de calcium (80g) et de chloroacétamide (149,6g,1,6 mole) dans 350 ml d'Eau/Ethanol (1 :1) est chauffé au reflux pendant 42 heures. Filtrer à chaud et laisser refroidir, essorer les cristaux et les ré-empâter à l'eau puis laver à l'éthanol absolu. Après séchage, on obtient 87,9 g de produit brut. Après deux recristallisations dans l'acétonitrile, on obtient 45,3 g de produit pur (Fusion = 178°C).

### Etape 2 : Préparation de 2-(4-Amino-phénylamino)-acétamide, dichlorhydrate (4)

Dans un hydrogénateur inox de 1L, charger 29,3 g (0,15 mole) 2-(4-Nitro-phenylamino)-acetamide (3), 500 ml d'éthanol absolu et ajouter 1 g de Pd/C (10% humide).

Le mélange est mis sous pression de 10 bars d'hydrogène à 50°C pendant 4 heures. Après filtration du catalyseur, on acidifie le filtrat avec 84 ml (0,36 mole) d'éthanol chlorhydrique 4,3 N. Diluer avec 84 ml d'éther isopropylique, essorer le précipité formé, laver à l' éther isopropylique puis sécher à 55°C. La masse du produit obtenu est 12,2 g.

| | **THEORIE, 2 HCl** | **TROUVE** |
|---|---|---|
| **C** | 40.33 | 32.49 |
| **H** | 5.46 | 7.22 |
| **N** | 17.63 | 17.50 |
| **Cl** | 29.78 | 30.12 |

Les spectres RMN du proton et de masse sont conformes à la structure attendue du produit.

### Exemple 3 : Synthèse du N-[3-(4-Methyl-piperazin-1-yl)-propyl]-benzene-1,4-diamine, dichlorhydrate (10)

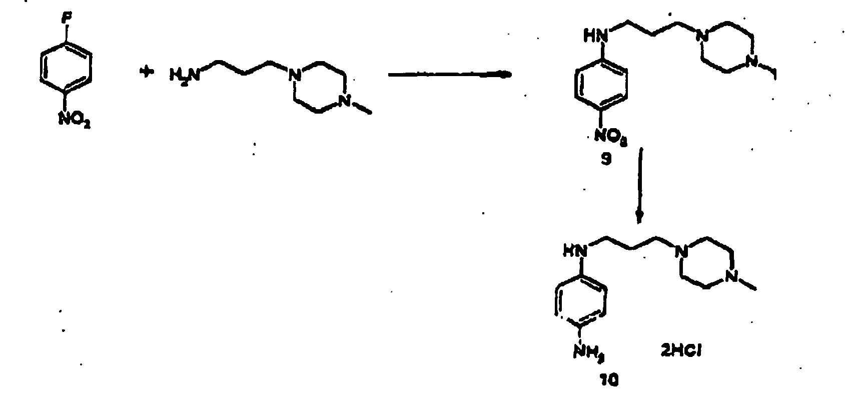

### Etape 1: synthèse du N-[3-(4-Methyl-piperazin-1-yl)-propyl]-4-nitro-1-aminobenzene (9)

A une solution de 20 ml de N-méthyl-pyrrolidinone, 2,67 g de N-(3-aminopropyl)-N'-méthylpipérazine et 2,35 g de K₂CO₃, on ajoute 2 g de 4-fluoro nitrobenzène. Le milieu réactionnel est chauffé à 60°C pendant 10 heures, puis, après refroidissement à la température ambiante, est versé dans un mélange eau + glace. Le précipité jaune formé est filtré, ré-empaté dans l'eau puis séché sur P₂O₅. On obtient 3,22 g de N-[3-(4-Methyl-piperazin-1-yl)-propyl]-4-nitro-1-aminobenzene (9).

### Etape 2: synthèse du N-[3-(4-Methyl-piperazin-1-yl)-propyl]-benzene-1,4-diamine, dichlorhydrate (10)

Le N-[3-(4-Methyl-piperazin-1-yl)-propyl]-4-nitro-1-aminobenzene (9) précédemment obtenu est réduit avec un mélange zinc / chlorure d'ammonium/Eau/ Ethanol bouillant. L'amine correspondante est isolée sous forme de dichlorhydrate.

Les spectres RMN du proton et de masse sont conformes à la structure attendue du produit.

### Example 4: Synthèse du 1-[3-(4-Amino-phenylamino)-propyl]-pyrrolidin-2-one, dichlorhydrate (12)

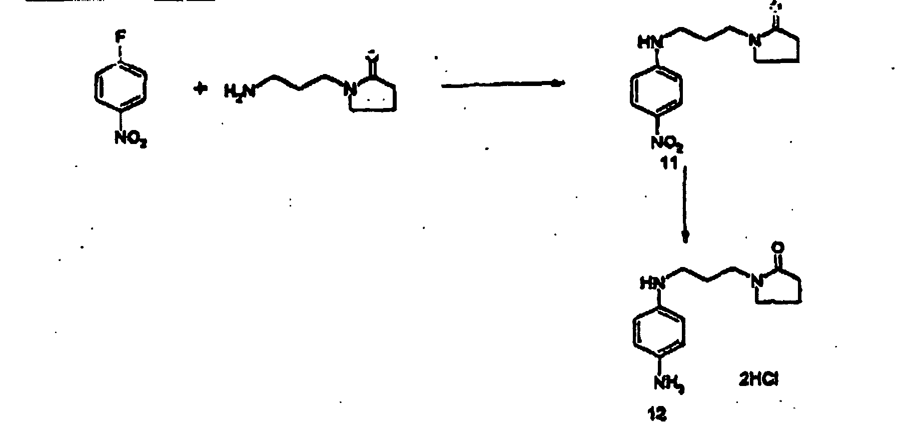

### Etape 1 : synthèse du 1-[3-(4-nitro-3-phenylamino)-propyl]-pyrrolidin-2-one (11)

A une solution de 20 ml de N-méthyl-pyrrolidinone, 2,41 g de N-(3'-aminopropyl)-2-pyrrolidinone et 1,72 g de triéthylamine on ajoute 2 g de 4-fluoro nitrobenzène. Le milieu réactionnel est chauffé à 60°C pendant 10 heures, puis, après refroidissement à la température ambiante, est versé dans un mélange eau + glace. Le précipité jaune formé est filtré, ré-empaté dans l'eau puis séché sur P₂O₅. On obtient 2,8 g de 1-[3-(4-nitro-3-phenylamino)-propyl]-pyrrolidin-2-one (11).

### Etape 2: synthèse du 1-[3-(4-Amino-3-phenylamino)-propyl]-pyrrolidin-2-one dichlorhydrate (12)

Le 1-[3-(4-nitro-3-phenylamino)-propyl]-pyrrolidin-2-one (11) précédemment obtenu est réduit avec un mélange zinc / chlorure d'ammonium/Eau/ Ethanol bouillant. L'amine correspondante est isolée sous forme de dichlorhydrate.

Les spectres RMN du proton et de masse sont conformes à la structure attendue du produit.

### Exemple 5: Synthèse du N-(3-Pyrrolidin-1-yl-propyl)-benzene-1,4,-diamine, dichlorhydrate (14)

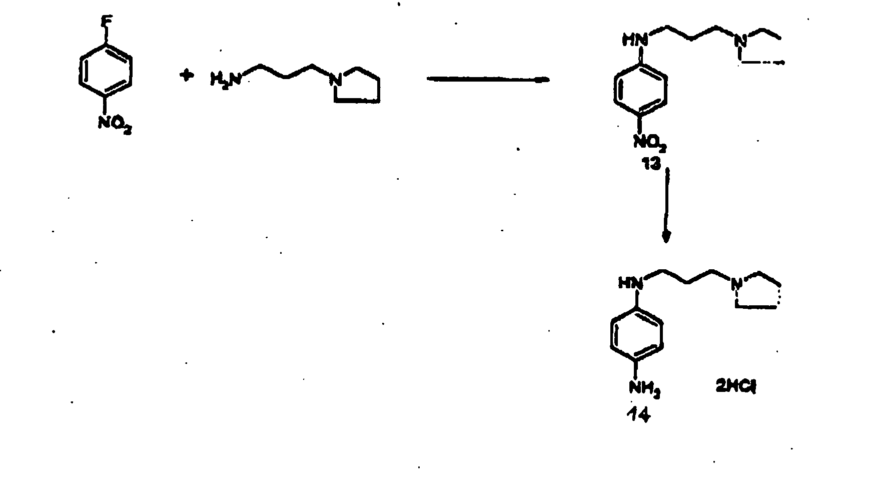

### Etape 1 : synthèse du N-(3-Pyrrolidin-1-yl-propyl)-4-nitro-1-aminobenzene (14)

A une solution de 20 ml de N-méthyl-pyrrolidinone, 2,18 g de N-(3-aminopropyl)pyrrolidine et 2,35 g de K₂CO₃, on ajoute 2 g de 4-fluoro nitrobenzène. Le milieu réactionnel est chauffé à 60°C pendant 12 heures, puis, après refroidissement à la température ambiante, est versé dans un mélange eau + glace. Le précipité jaune formé est filtré, ré-empaté dans l'eau puis séché sur P₂O₅. On obtient 3,3 g de N-(3-Pyrrolidin-1-yl-propyl)-4-nitro-1-aminobenzene (14)

### Etape 2 : synthèse du N-(3-Pyrrolidin-1-yl-propyl)-benzene-1,4-diamine, dichlorhydrate (15)

Le N-(3-Pyrrolidin-1-yl-propyl)-4-nitro-1-aminobenzene (14) précédemment obtenu est réduit avec un mélange zinc / chlorure d'ammonium/Eau/ Ethanol bouillant. L'amine correspondante est isolée sous forme de dichlorhydrate.

Les spectres RMN du proton et de masse sont conformes à la structure attendue du produit.

### Exemple 6: Synthèse du N-(3-Pyrrolidin-1-yl-ethyl)-benzene-1,4-diamine, dichlorhydrate (16)

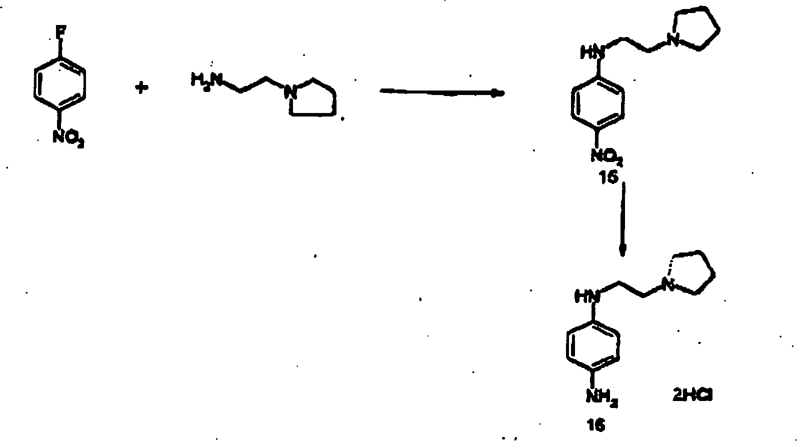

### Etape 1 : synthèse du N-(3-Pyrrolidin-1-yl-ethyl)-4-nitro-1-aminobenzene (15)

A une solution de 20 ml de N-méthyl-pyrrolidinone, 1,94 g de 2-(1-pyrrolidino)éthylamine et 2,35 g de K₂CO₃, on ajoute 2 g de para-fluoro-nitrobenzène. Le milieu réactionnel est chauffé à 60°C pendant 8 heures, puis, après refroidissement à la température ambiante, est versé dans un mélange eau + glace. Le précipité jaune formé est filtré, ré-empaté dans l'eau puis séché sur P₂O₃. On obtient 2,5 g de N-(3-Pyrrolidin-1-yl-ethyl)-4-nitro-1-aminobenzen (15).

### Etape 2: synthèse du N-(3-Pyrrolidin-1-yl-ethyl)-benzene-1,4-diamine dichlorhydrate (16)

Le N-(3-Pyrrolidin-1-yl-ethyl)-4-nitro-1-aminobenzene (15) précédemment obtenu est réduit avec un mélange zinc / chlorure d'ammonium/Eau/ Ethanol bouillant. L'amine correspondante est isolée sous forme de dichlorhydrate.

Les spectres RMN du proton et de masse sont conformes à la structure attendue du produit.

### Exemple 7: Synthèse du N-[3-(2-Methyl-piperidin-1-yl)-propyl]-benzene-1,4-diamine, dichlorhydrate

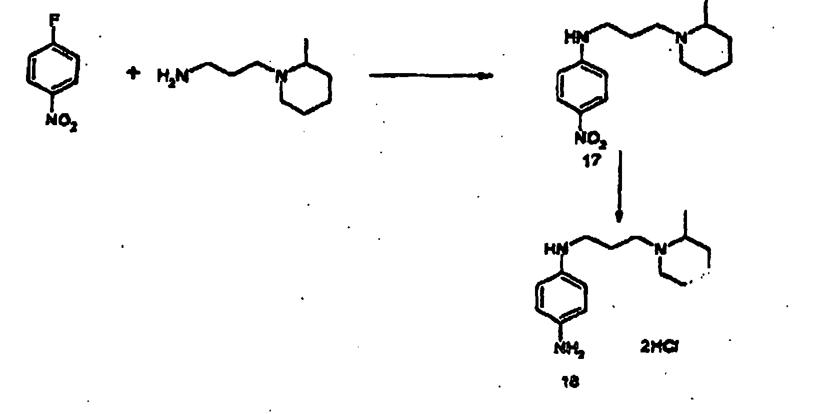

### Etape 1: synthèse du N-[3-(2-Methyl-piperidin-1-yl)-propyl]-4-nitro-1-aminobenzene (17)

A une solution de 20 ml de N-méthyl-pyrrolidinone, 1,65 g de 1-(3-aminopropyl)-2-pipécoline et 1,72 g de triéthylamine on ajoute 2 g de para-fluoro-nitrobenzène. Le milieu réactionnel est chauffé à 60°C pendant 8 heures, puis, après refroidissement à la température ambiante, est versé dans un mélange eau + glace. Le précipité jaune formé est filtré, ré-empaté dans l'eau puis séché sur P₂O₅. On obtient 2,2 g de N-[3-(2-Methyl-piperidin-1-yl)-propyl]-4-nitro-1-aminobenzene (17).

### Etape 2 : synthèse du N-[3-(2-Methyl-piperidin-1-yl)-propyl]-benzene-1,4-diamine, dichlorhydrate (18)

Le N-[3-(2-Methyl-piperidin-1-yl)-propyl]-4-nitro-1-aminobenzene (17) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 8: Synthèse du N-(3-(2-[2-(3-aminopropoxy)ethoxylethoxy}propyl) benzene-1,4-diamine hydrochloride

### Etape 1: préparation du N-(3-{2-[2-(3-aminopropoxy)ethoxy]ethoxy}propyl) N-(4-nitrophenyl)amine

Dans un tricot, on introduit sous azote 1,41 g (0,01 mole) de 1-fluoro-4-nitrobenzène et 1,62 ml (0,02 mole) de pyridine. On ajoute goutte-à-goutte avec agitation 8,75 ml (0,04 mole) de 4,7,10-trioxatridécane-1,13-diamine. On chauffe jusqu'au 70 °C. Après 24 heures de réaction, le mélange réactionnel est refroidit puis 40 ml d'eau distillée est ajouté avec forte agitation. Ensuite, le mélange réactionnel est extrait au dichlorométhane, lavé à l'eau 2 fois, séché sur MgSO₄ puis évaporé sous vide. Le produit brut isolé sous forme d'une huile jaune contenant d'environs 90% de dérivé mono-nitré attendu est purifié sur une colonne de silice, éluant : acétate d'éthyle (pour séparer le dérivé dinitré) puis le méthanol (pour récupérer le dérivé mono-nitré attendu). On obtient 1,8 g de produit attendu sous forme d'une huile jaune foncée.

### Etape 2: préparation du N-(3-{2-[2-(3-aminopropoxy)ethoxy]ethoxy}propyl) benzene-1,4-diamine hydrochloride

Dans un autoclave (hydrogénateur) de 200 ml, muni d'un agitateur magnétique, on introduit 1,5 g de dérivé nitré préparé précédemment et environs 100 ml de méthanol. La solution obtenue est dégazée à l'azote. On y ajoute 0,4 g de palladium sur charbon (5% humide à 50% d'eau). On agite le mélange réactionnel, en purgeant une fois à l'hydrogène puis introduit l'hydrogène sous pression d'environ 5 bar. Après 4 heures de réaction, le recteur est purgée à l'azote mélange réactionnel est filtré rapidement sur célite sous une pression légère d'azote. Le filtrat est récupéré dans une solution préalablement refroidit de méthanol contenant environs 3 équivalents d'acide chlorhydrique gazeux. On rince plusieurs fois avec le méthanol sous un courant d'azote. La solution ainsi obtenue concentrée est ensuite traitée avec l'éther, Le produit obtenu sous forme d'une pâte rose claire est agité puis rincé plusieurs fois avec l'acétonitrile et de l'éther sous azote. 1,6 g de produit attendu est isolé sous forme d'une poudre blanche légèrement rose.
Les spectres de RMN du proton et du ¹³C et de microanalyses sont conformes à la structure attendu du produit.

### EXEMPLES DE FORMULATION

### Exemples 1 à 6 : Composition tinctoriale à partir du 2-(4-Amino-phénylamino)-acétamide, dichlorhydrate (4)

### -Exemples 1 à 3 : Teinture en milieu acide

Les compositions tinctoriales suivantes sont préparées :

| **Exemple** | **1** | **2** | **3** |
|---|---|---|---|
| 2-(4-Amino-phenylamino)-acetamide, dichlorhydrate (4) | 10-3 mole | 10-3 mole | 10-3 mole |
| 1H-Indol-6-ol | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture (1) | (*) | (*) | (*) |
| Eau déminéralisée q.s.p. | 100g | 100g | 100g |

| | | | |
|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Example** | **1** | **2** | **3** |
|---|---|---|---|
| **Nuance observée** | brun orange | gris | rouge |

### - Exemples 4 à 6 : teinture en milieu basique

Les compositions tinctoriales suivantes sont préparées :

| **Exemple** | **4** | **5** | **6** |
|---|---|---|---|
| 2-(4-Amino-phenylamino)-acetamide, dichlorhydrate (4) | 10-3 mole | 10-3 mole | 10-3 mole |
| 3,6-Dimethyl-1H-pyrazolo[5,1-c][1,2,4]triazole | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture(2) | (*) | (*) | (*) |
| Eau déminéralisée q.s.p. | 100g | 100g | 100g |

| | | | |
|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous:

| **Example** | **4** | **5** | **6** |
|---|---|---|---|
| **Nuance observée** | Rouge chromatique | gris violet-bleu | violet-rouge |

### Exemples 17 à 26 : Composition tinctoriale à partir du 1-[3-(4-Amino-phenylamino)-propyl]-pyrrolidin-2-one, dichlorhydrate (12)

### - Exemples 17 à 23: Teinture en milieu acide

Les compositions tinctoriales suivantes sont préparées :

| Exemple | 17 | 18 | 19 | 20 | 21 | 22 | 23 |
|---|---|---|---|---|---|---|---|
| 1-[3-(4-Amino-phenylamino)-propyl]-pyrrolidin-2-one, dichlorhydrate (12) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1 H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na2HPO4 0,28 g KH2PO4 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **17** | **18** | **19** | **20** | **21** | **22** | **23** |
|---|---|---|---|---|---|---|---|
| Nuance observée | brun | gris violet intense | brun rouge intense | brun rouge intense | brun rouge | gris bleu intense | violet-bleu intense |

### - Exemples 24 à 26 : teinture en milieu basique

Les compositions tinctoriales suivantes sont préparées :

| **Exemple** | **24** | **25** | **26** |
|---|---|---|---|
| 1-[3-(4-Amino-phenylamino)-propyl]-pyrrolidin-2-one, dichlorhydrate (12) | 10-3 mole | 10-3 mole | 10-3 mole |
| 5-Amino-2-methyl-phenol | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture (2) | (*) | (*) | (*) |
| Eau déminéralisée q.s.p. | 100g | 100g | 100g |

| | | | |
|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **24** | **25** | **26** |
|---|---|---|---|
| Nuance observée | gris violet-rouge | bleu intense | violet-bleu intense |

### Exemples 27 à 38 : Composition tinctoriale à partir du N-(3-Pyrrolidin-1-yl-propyl)-benzene-1,4-diamine, dichlorhydrate (14)

### - Exemples 27 à 33 : Teinture en milieu acide

Les compositions tinctoriales suivantes sont préparées :

| **Exemple** | **27** | **28** | **29** | **30** | **31** | **32** | **33** |
|---|---|---|---|---|---|---|---|
| N-(3-Pyrrolidin-1-yl-propyl)-benzene-1,4-diamine, dichlorhydrate (14) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **27** | **28** | **29** | **30** | **31** | **32** | **33** |
|---|---|---|---|---|---|---|---|
| Nuance observée | brun | gris violet-rouge | brun rouge intense | brun rouge | brun orangé | gris bleu intense | violet intense |

### - Exemples 34 à 38 : teinture en milieu basique

Les compositions tinctoriales suivantes sont préparées :

| **Exemple** | **34** | **35** | **36** | **37** | **38** |
|---|---|---|---|---|---|
| N-(3-Pyrrolidin-1-yl-propyl)-benzene-1,4-diamine, dichlorhydrate (14) | 10-3 mole | 10-3 mole | 10-3 mole | 10-3 mole | 10-3 mole |
| 5-Amino-2-methyl-phenol | 10-3 mole | | | | |
| 2-Amino-pyridin-3-ol | | 10-3 mole | | | |
| 3,6-Dimethyl-1H-pyrazolo[5,1-c][1,2 ,4]triazole | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*): support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **34** | **35** | **36** | **37** | **38** |
|---|---|---|---|---|---|
| Nuance observée | violet-rouge | rouge | rouge chromatique | bleu intense | violet intense |

### Exemples 39 à 47 : Composition tinctoriale à partir du N-(3-Pyrrolidin-1-yl-ethyl)-benzene-1,4-diamine, dichlorhydrate (16)

### - Exemples 39 à 42 : Teinture en milieu acide

Les compositions tinctoriales suivantes sont préparées :

| **Exemple** | **39** | **40** | **41** | **42** |
|---|---|---|---|---|
| N-(3-Pyrrolidin-1-yl-ethyl)-benzene-1,4-diamine, dichlorhydrate (16) | 10-3 mole | 10-3 mole | 10-3 mole | 10-3 mole |
| 1H-Indol-6-ol | 10-3 mole | | | |
| 2-Amino-pyridin-3-ol | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (1) | (*) | (*) | (*) | (*) |
| Eau déminéralisée q.s.p. | 100g | 100g | 100g | 100g |

| | | | | |
|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **39** | **40** | **41** | **42** |
|---|---|---|---|---|
| Nuance observée | brun rouge intense | orangé | gris bleu intense | violet-rouge |

### - Exemples 43 à 47 : teinture en milieu basique

Les compositions tinctoriales suivantes sont préparées :

| **Exemple** | **43** | **44** | **45** | **46** | **47** |
|---|---|---|---|---|---|
| N-(3-Pyrrolidin-1-yl-ethyl)-benzene-1,4-diamine, dichlorhydrate (16) | 10-3 mole | 10-3 mole | 10-3 mole | 10-3 mole | 10-3 mole |
| 5-Amino-2-methyl-phenol | 10-3 mole | | | | |
| 2-Amino-pyridin-3-ol | | 10-3 mole | | | |
| 3,6-Dimethyl-1H-pyrazolo[5,1-c][1,2 ,4]triazole | | | 10-3 mole | | |
| 2-(2,4-Diamino-phenoxy)-ethanol,chlorhydrate | | | | 10-3 mole | |
| 3-Amino-2-chloro-6-methyl-phenol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **43** | **44** | **45** | **46** | **47** |
|---|---|---|---|---|---|
| Nuance observée | rouge | orangé | rouge chromatique | violet-bleu intense | violet intense |

### Exemples 48 à 58 : Composition tinctoriale à partir du N-[3-(4-Methyl-piperazin-1-yl)-propyl]-benzene-1,4-diamine, dichlorhydrate (10)

### - Exemples 48 à 54 : Teinture en milieu acide

Les compositions tinctoriales suivantes sont préparées :

| **Exemple** | **48** | **49** | **50** | **51** | **52** | **53** | **54** |
|---|---|---|---|---|---|---|---|
| N-[3-(4-Methyl-piperazin-1-yl)-propyl]-benzene-1,4-diamine, dichlorhydrate (10) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **48** | **49** | **50** | **51** | **52** | **53** | **54** |
|---|---|---|---|---|---|---|---|
| Nuance observée | brun jaune | gris violet-rouge intense | brun rouge intense | brun rouge | brun orangé | bleu intense | violet-bleu intense |

### - Exemples 55 à 58 : teinture en milieu basique

Les compositions tinctoriales suivantes sont préparées :

| **Exemple** | **55** | **56** | **57** | **58** |
|---|---|---|---|---|
| N-[3-(4-Methyl-piperazin-1-yl)-propyl]-benzene-1,4-diamine, dichlorhydrate (10) | 10-3 mole | 10-3 mole | 10-3 mole | 10-3 mole |
| 5-Amino-2-methyl-phenol | 10-3 mole | | | |
| 3,6-Dimethyl-1H-pyrazolo[5,1-c][1,2 ,4]triazole | | 10-3 mole | | |
| 2-(2,4-Diamino-phenoxy)-ethanol,ch lorhydrate | | | 10-3 mole | |
| 3-Amino-2-chloro-6-methyl-phenol,chlorhydrate | | | | 10-3 mole |
| Support de teinture (2) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **55** | **56** | **57** | **58** |
|---|---|---|---|---|
| Nuance observée | gris rouge | rouge chronique | bleu | violet-bleu intense |

### Exemples 59 à 68 : Composition tinctoriale à partir du N-(3-Pyrrolidin-1-yl-ethyl)-benzene-1,4-diamine, dichlorhydrate (16)

### - Exemples 59 à 64 : Teinture en milieu acide

Les compositions tinctoriales suivantes sont préparées :

| **Exemple** | **59** | **60** | **61** | **62** | **63** | **64** |
|---|---|---|---|---|---|---|
| N-(3-Pyrrolidin-1-yl-ethyl)-benzene-1,4-diamine, dichlorhydrate (16) | 10-3 mole | 10-3 mole | 10-3 mole | 10-3 mole | 10-3 mole | 10-3 mole |
| Benzene-1,3-diol | 10-3 mole | | | | | |
| 5-Amino-2-methyl-phenol | | 10-3 mole | | | | |
| 1H-Indol-6-ol | | | 10-3 mole | | | |
| 3,6-Dimethyl-1H-pyrazolo[5,1-c][1,2 ,4]triazole | | | | 10-3 mole | | |
| 2-(2,4-Diamino-phenoxy)-ethanol,chlorhydrate | | | | | 10-3 mole | |
| 3-Amino-2-chloro-6-methyl-phenol,chlorhydrate | | | | | | 10-3 mole |
| Support de teinture (1) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (1) pH 7 | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **59** | **60** | **61** | **62** | **63** | **64** |
|---|---|---|---|---|---|---|
| Nuance observée | brun | violet-rouge intense | brun intense | brun orangé | gris bleu intense | violet intense |

### - Exemples 65 à 68 : teinture en milieu basique

Les compositions tinctoriales suivantes sont préparées :

| **Exemple** | **65** | **66** | **67** | **68** |
|---|---|---|---|---|
| N-(3-Pyrrolidin-1-yl-ethyl)-benzene-1,4-diamine, dichlorhydrate (16) | 10-3 mole | 10-3 mole | 10-3 mole | 10-3 mole |
| Benzene-1,3-diol | 10-3 mole | | | |
| 5-Amino-2-methyl-phenol | | 10-3 mole | | |
| 2-(2,4-Diamino-phenoxy)-ethanol,chlorhydrate | | | 10-3 mole | |
| 3-Amino-2-chloro-6-methyl-phenol,chlorhydrate | | | | 10-3 mole |
| Support de teinture (2) | (*) | (*) | (*) | (*) |
| Eau déminéralisée q.s.p. | 100g | 100g | 100g | 100g |

| | | | | |
|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **65** | **66** | **67** | **68** |
|---|---|---|---|---|
| Nuance observée | brun | gris rouge | gris bleu intense | violet intense |

## Revendications

1. Composé **caractérisé par le fait qu'**il s'agit d'une para-phénylènediamine secondaire N-alkylaminée de formule générale (I) : 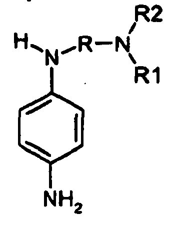 dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle,
et sous réserve que le composé de formule (I) ne soit pas le 4-[4-(pipéridinyl)butylamino]aminobenzène, ni la N-[3-(4-méthylpipérazin-1-yl)propyl]benzène 1,4-diamine, ni le 4-[2-(pipéridinyl)éthylamino]aminobenzène.

2. Composé de formule (I) selon la revendication 1 dans laquelle R₁ représente un atome d'hydrogène, R₂ étant tel que défini à la revendication 1.

3. Composé de formule (I) selon la revendication 1 dans laquelle R₁ et R₂ forment ensemble avec l'azote qu'ils substituent un cycle saturé à 5 ou 6 éventuellement interrompu par un ou plusieurs atomes d'azote ou par une ou plusieurs fonctions carbonyles, et éventuellement substitué par un ou plusieurs groupements alkyles.

4. Composé selon la revendication 3 dans laquelle R₁ et R₂ forment ensemble avec l'azote qu'ils substituent un cycle saturé à 5 ou 6 chaînons interrompu par un ou plusieurs atomes d'azote ou par une fonction carbonyle, et éventuellement substitué par un groupement alkyle en C₁-C₃ .

5. Composé de formule (I) selon la revendication 1 dans laquelle R représente un radical alkylène linéaire en C₁-C₇, de préférence C₁-C₃.

6. Composé de formule (I) selon la revendication 5 dans laquelle R est non substitué ou interrompu par un ou plusieurs atomes choisis parmi l'azote et l'oxygène.

7. Composé de formule (I) selon l'une des revendications précédentes **caractérisé par le fait qu'**il est choisi parmi la N-[3-(3-aminopropylamino)-propyl]benzène-1,4-diamine ; N-(2-pyrrolidin-1-yl-éthyl)-benzène-1,4 diamine ; N-[3-(2-méthylpipéridin-1-yl)-propyl]benzène-1,4-diamine ; N-(3-pyrrolidin-1-yl-propyl)-benzène-1,4 diamine ; N-[3-(4-méthylpipérazin-1-yl)-propyl]benzène-1,4-diamine ; 1-[3-(4-aminophénylamino)-propyl]-pyrrolidin-2-one, N-{2-[2-(2-diméthylaminoéthoxy)-éthoxy]-éthyl}benzène-1,4-diamine.

8. Composé de formule (I) selon l'une des revendications précédentes **caractérisé par le fait qu'**il se présente sous la forme d'un sel.

9. Composé de formule (I) selon la revendication 8 **caractérisé par le fait qu'**il se présente sous la forme d'un sel d'addition avec un acide choisi parmi les chlorhydrates, les bromhydrates, les sulfates, les citrates, les succinates, les tartrates, les lactates, les tosylates, les benzènesulfonates, les phosphates et les acétates

10. Préparation du composé selon l'une des revendications 1 à 9 **caractérisé par le fait qu'**on effectue une réduction du composé nitré correspondant.

11. Utilisation d'au moins un composé de formule générale (I) selon l'une des revendications 1 à 9 dans laquelle les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène ou par une ou plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle, pour la teinture des fibres, de préférence les fibres kératiniques telles que les cheveux.

12. Composition cosmétique pour la teinture des fibres kératiniques, de préférence les fibres kératiniques humaines telles que les cheveux, comprenant, dans un milieu approprié pour la teinture, au moins un composé de formule générale (I) selon l'une des revendications 1 à 9 dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène ou par une ou plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle.

13. Composition selon la revendication 12 **caractérisée par le fait qu'**elle comprend au moins un adjuvant cosmétique choisi dans le groupe formé par les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

14. Composition cosmétique pour la teinture des fibres, de préférence les fibres kératiniques humaines telles que les cheveux, comprenant, dans un milieu approprié pour la teinture, au moins un composé de formule générale (I) selon l'une des revendications 1 à 9
et au moins un adjuvant cosmétique choisi dans le groupe formé par les agents anti-oxydants, les agents de pénétration, les agents séquestrants, les parfums, les tampons, les agents dispersants, les tensioactifs, les agents de conditionnement, les agents filmogènes, les polymères, les céramides, les agents conservateurs, les agents nacrants ou opacifiants, les vitamines ou provitamines.

15. Composition selon l'une des revendications 12 à 14 **caractérisée par le fait que** la concentration du composé de formule générale (I) est comprise entre 0,0001 et 20%, de préférence entre 0,005 à 6% en poids par rapport au poids total de la composition.

16. Composition selon l'une des revendications 12 à 15 **caractérisée par le fait que** le milieu approprié pour la teinture est constitué par de l'eau ou par un mélange d'eau et d'au moins un solvant organique tel que les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

17. Composition selon l'une des revendications 12 à 16 **caractérisée par le fait que** la quantité en adjuvant cosmétique est comprise pour chacun d'eux entre 0,01 et 20 % en poids par rapport au poids de la composition.

18. Composition selon l'une des revendications 12 à 17 **caractérisée par le fait qu'**elle comprend au moins un précurseur de colorant d'oxydation additionnel différent des composés de formule (I) selon l'une des revendications 1 à 9.

19. Composition selon la revendication 18 **caractérisée par le fait que** le précurseur de colorant d'oxydation additionnel est un coupleur d'oxydation classique choisi parmi les méta-phénylènediamines, les méta-aminophénols, les méta-diphénols, les coupleurs naphtaléniques, les coupleurs hétérocycliques ainsi que leurs sels d'addition.

20. Composition selon la revendication 19 **caractérisée par le fait que** la concentration de ce coupleur est comprise entre 0,0001 et 20 % en poids par rapport au poids total de la composition.

21. Composition selon l'une des revendications 17 à 20 **caractérisée par le fait que** le précurseur de colorant d'oxydation est une base d'oxydation différente des composés de composés de formule (I) selon l'une des revendications 1 à 9 choisie parmi les para-phénylènediamines, les bis-phénylalkylènediamines, les para-aminophénols, les ortho-aminophénols, les bases hétérocycliques et leurs sels d'addition.

22. Composition selon la revendication 21 telle que la concentration de cette base d'oxydation est comprise entre 0,0001 et 20% en poids par rapport au poids total de la composition.

23. Composition selon l'une des revendications 12 à 22 **caractérisée par le fait qu'**elle renferme un ou plusieurs colorants directs naturels ou cationiques.

24. Composition prête à l'emploi **caractérisée par le fait qu'**il s'agit d'un mélange d'une composition selon l'une des revendications 12 à 23 avec au moins un agent oxydant choisi parmi le peroxyde d'hydrogène, le peroxyde d'urée, les bromates de métaux alcalins, les persels, les peracides et les enzymes oxydases.

25. Procédé de teinture des fibres kératiniques et en particulier des fibres kératiniques humaines telles que les cheveux, **caractérisée par le fait qu'**on applique sur lesdites fibres au moins une composition selon l'une des revendications 12 à 23, pendant une durée suffisante pour développer la coloration désirée en présence d'un agent oxydant, l'agent oxydant étant appliqué avant, simultanément ou après la composition.

26. Procédé de teinture des fibres kératiniques et en particulier des fibres kératiniques humaines telles que les cheveux, **caractérisée par le fait qu'**on applique sur lesdites fibres la composition prête à l'emploi selon la revendication 24, pendant une durée suffisante pour développer la coloration désirée

27. Utilisation d'une composition comprenant, dans un milieu approprié pour la teinture, au moins un composé de formule générale (I) telle que défini à la revendication 1, dans laquelle :
les radicaux R₁ et R₂ représentent, indépendamment l'un de l'autre, un atome d'hydrogène, un groupement alkyle, un groupement monoalkylaminocarbonyle, un groupement dialkylaminocarbonyle, ou les radicaux R₁ et R₂ forment, ensemble et avec l'azote qu'ils substituent, un cycle saturé à 5 ou 6 chaînons éventuellement interrompu par un ou plusieurs atomes d'azote ou par une ou plusieurs fonctions carbonyles, et éventuellement substitué par un ou plusieurs groupements alkyles,
le radical R représente un radical alkylène en C₁-C₁₀, linéaire ou ramifié, éventuellement interrompu par un ou plusieurs atomes choisis parmi l'azote et l'oxygène ou par une ou plusieurs fonctions carbonyle, et éventuellement substitué par un ou plusieurs groupements choisis parmi les groupements amino, monoalkylamino, dialkylamino, alkylcarbonyle, amido, monoalkylaminocarbonyle, dialkylaminocarbonyle,
sous réserve que, lorsque R représente un radical alkylène en C₁-C₁₀, linéaire ou ramifié, non substitué et non interrompu par un ou plusieurs atomes choisis parmi l'azote et l'oxygène ou par une ou plusieurs fonctions carbonyles, alors les radicaux R₁ et R₂ ne représentent pas l'un des couples suivants : deux atomes d'hydrogène, deux groupements alkyles, un atome d'hydrogène et un groupement alkyle pour la teinture des fibres, de préférence les fibres kératiniques telles que les cheveux.

28. Utilisation de la composition selon la revendication 27 dans un dispositif à plusieurs compartiments, un premier compartiment contenant la composition cosmétique pour la teinture des fibres kératiniques et un deuxième compartiment contenant un agent oxydant.

## Claims

1. Compound, **characterized in that** it is an N-alkylamino secondary para-phenylenediamine of general formula (I) : 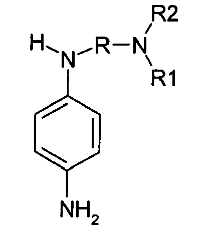 in which:
the radicals R₁ and R₂ represent, independently of each other, a hydrogen atom, an alkyl group, a monoalkylaminocarbonyl group or a dialkylaminocarbonyl group, or the radicals R₁ and R₂ form, together with the nitrogen on which they are substituent, a saturated 5- or 6-membered ring optionally interrupted with one or more nitrogen atoms or with one or more carbonyl functions, and optionally substituted with one or more alkyl groups,
the radical R represents a linear or branched C₁-C₁₀ alkylene radical, optionally interrupted with one or more atoms chosen from nitrogen and oxygen, and optionally substituted with one or more groups chosen from amino, monoalkylamino, dialkylamino, alkylcarbonyl, amido, monoalkylaminocarbonyl and dialkylaminocarbonyl groups,
with the proviso that, when R represents a linear or branched C₁-C₁₀ alkylene radical that is unsubstituted and not interrupted with one or more atoms chosen from nitrogen and oxygen or with one or more carbonyl functions, then the radicals R₁ and R₂ do not represent one of the following couples: two hydrogen atoms, two alkyl groups, a hydrogen atom and an alkyl group,
and with the proviso that the compound of formula (I) is not 4-[4-(piperidinyl)butylamino]aminobenzene or N-(3-(4-methylpiperazin-1-yl)propylbenzene-1,4-diamine or 4-[2-(piperidinyl)ethylamino]aminobenzene.

2. Compound of formula (I) according to Claim 1, in which R₁ represents a hydrogen atom, R₂ being as defined in Claim 1.

3. Compound of formula (I) according to Claim 1, in which R₁ and R₂ form, together with the nitrogen on which they are substituent, a saturated 5- or 6-membered ring optionally interrupted with one or more nitrogen atoms or with one or more carbonyl functions, and optionally substituted with one or more alkyl groups.

4. Compound according to Claim 3, in which R₁ and R₂ form, together with the nitrogen on which they are substituent, a saturated 5- or 6-membered ring interrupted with one or more nitrogen atoms or with a carbonyl function, and optionally substituted with a C₁-C₃ alkyl group.

5. Compound of formula (I) according to Claim 1, in which R represents a linear C₁-C₇ and preferably C₁-C₃ alkylene radical.

6. Compound of formula (I) according to Claim 5, in which R is unsubstituted or interrupted with one or more atoms chosen from nitrogen and oxygen.

7. Compound of formula (I) according to one of the preceding claims, **characterized in that** it is chosen from N-[3-(3-aminopropylamino)propyl]benzene-1,4-diamine; N-(2-pyrrolidin-1-ylethyl)benzene-1,4-diamine; N-[3-(2-methylpiperidin-1-yl)propyl]benzene-1,4-diamine; N-(3-pyrrolidin-1-ylpropyl)benzene-1,4-diamine; N-[3-(4-methylpiperazin-1-yl)propyl]benzene-1,4-diamine; 1-[3-(4-aminophenylamino)propyl]pyrrolidin-2-one; N-{2-[2-(2-dimethyl aminoethoxy)ethoxy]ethyl}benzene-1,4-diamine.

8. Compound of formula (I) according to one of the preceding claims, **characterized in that** it is in the form of a salt.

9. Compound of formula (I) according to Claim 8, **characterized in that** it is in the form of an addition salt with an acid chosen from the hydrochlorides, hydrobromides, sulfates, citrates, succinates, tartrates, lactates, tosylates, benzenesulfonates, phosphates and acetates.

10. Preparation of the compound according to one of Claims 1 to 9, **characterized in that** a reduction of the corresponding nitro compound is performed.

11. Use of at least one compound of general formula (I) according to one of Claims 1 to 9, in which the radicals R₁ and R₂ represent, independently of each other, a hydrogen atom, an alkyl group, a monoalkylaminocarbonyl group or a dialkylaminocarbonyl group, or the radicals R₁ and R₂ form, together with the nitrogen on which they are substituent, a saturated 5- or 6-membered ring optionally interrupted with one or more nitrogen atoms or with one or more carbonyl functions, and optionally substituted with one or more alkyl groups,
the radical R represents a linear or branched C₁-C₁₀ alkylene radical, optionally interrupted with one or more atoms chosen from nitrogen and oxygen or with one of more carbonyl functions, and optionally substituted with one or more groups chosen from amino, monoalkylamino, dialkylamino, alkylcarbonyl, amido, monoalkylaminocarbonyl and dialkylaminocarbonyl groups, with the proviso that, when R represents a linear or branched C₁-C₁₀ alkylene radical that is unsubstituted and not interrupted with one or more atoms chosen from nitrogen and oxygen or with one or more carbonyl functions, then the radicals R₁ and R₂ do not represent one of the following couples: two hydrogen atoms, two alkyl groups, a hydrogen atom and an alkyl group, for dyeing fibres, preferably keratin fibres such as the hair.

12. Cosmetic composition for dyeing keratin fibres, preferably human keratin fibres such as the hair, comprising, in a medium that is suitable for dyeing, at least one compound of general formula (I) according to one of Claims 1 to 9, in which:
the radicals R₁ and R₂ represent, independently of each other, a hydrogen atom, an alkyl group, a monoalkylaminocarbonyl group or a dialkylaminocarbonyl group, or the radicals R₁ and R₂ form, together with the nitrogen on which they are substituent, a saturated 5- or 6-membered ring optionally interrupted with one or more nitrogen atoms or with one or more carbonyl functions, and optionally substituted with one or more alkyl groups,
the radical R represents a linear or branched C₁-C₁₀ alkylene radical, optionally interrupted with one or more atoms chosen from nitrogen and oxygen or with one or more carbonyl functions, and optionally substituted with one or more groups chosen from amino, monoalkylamino, dialkylamino, alkylcarbonyl, amido, monoalkylaminocarbonyl and dialkylaminocarbonyl groups, with the proviso that, when R represents a linear or branched C₁-C₁₀ alkylene radical that is unsubstituted and not interrupted with one or more atoms chosen from nitrogen and oxygen or with one or more carbonyl functions, then the radicals R₁ and R₂ do not represent one of the following couples: two hydrogen atoms, two alkyl groups, a hydrogen atom and an alkyl group.

13. Composition according to Claim 12, **characterized in that** it comprises at least one cosmetic adjuvant chosen from the group formed by antioxidants, penetrating agents, sequestering agents, fragrances, buffers, dispersants, surfactants, conditioning agents, film-forming agents, polymers, ceramides, preserving agents, nacreous agents or opacifiers, and vitamins or provitamins.

14. Cosmetic composition for dyeing fibres, preferably human keratin fibres such as the hair, comprising, in a medium that is suitable for dyeing, at least one compound of general formula (I) according to one of Claims 1 to 9 and at least one cosmetic adjuvant chosen from the group formed by antioxidants, penetrating agents, sequestering agents, fragrances, buffers, dispersants, surfactants, conditioning agents, film-forming agents, polymers, ceramides, preserving agents, nacreous agents or opacifiers, and vitamins or provitamins.

15. Composition according to one of Claims 12 to 14, **characterized in that** the concentration of the compound of general formula (I) is between 0.0001% and 20% and preferably between 0.005% and 6% by weight relative to the total weight of the composition.

16. Composition according to one of Claims 12 to 15, **characterized in that** the medium that is suitable for dyeing consists of water or of a mixture of water and of at least one organic solvent such as branched or unbranched C₁-C₄ lower alcohols, such as ethanol and isopropanol; polyols and polyol ethers, for instance 2-butoxyethanol, propylene glycol, propylene glycol monomethyl ether, diethylene glycol monomethyl ether and monoethyl ether and glycerol, and also aromatic alcohols, for instance benzyl alcohol or phenoxyethanol, and mixtures thereof.

17. Composition according to one of Claims 12 to 16, **characterized in that** the amount of cosmetic adjuvant is, for each of them, between 0.01% and 20% by weight relative to the weight of the composition.

18. Composition according to one of Claims 12 to 17, **characterized in that** it comprises at least one additional oxidation dye precursor other than the compounds of formula (I) according to one of Claims 1 to 9.

19. Composition according to Claim 18, **characterized in that** the additional oxidation dye precursor is a conventional oxidation coupler chosen from meta-phenylenediamines, meta-aminophenols, meta-diphenols, naphthalene-based couplers and heterocyclic couplers, and also the addition salts thereof.

20. Composition according to Claim 19, **characterized in that** the concentration of this coupler is between 0.0001% and 20% by weight relative to the total weight of the composition.

21. Composition according to one of Claims 17 to 20, **characterized in that** the oxidation dye precursor is an oxidation base other than the compounds of formula (I) according to one of Claims 1 to 9, chosen from para-phenylenediamines, bis(phenyl)alkylenediamines, para-aminophenols, ortho-aminophenols and heterocyclic bases, and the addition salts thereof.

22. Composition according to Claim 21, **characterized in that** the concentration of this oxidation base is between 0.0001% and 20% by weight relative to the total weight of the composition.

23. Composition according to one of Claims 12 to 22, **characterized in that** it contains one or more natural or cationic direct dyes.

24. Ready-to-use composition, **characterized in that** it is a mixture of a composition according to one of Claims 12 to 23 with at least one oxidizing agent chosen from hydrogen peroxide, urea peroxide, alkaline metal bromates, persalts, peracids and oxidase enzymes.

25. Process for dyeing keratin fibres, and in particular human keratin fibres such as the hair, **characterized in that** at least one composition according to one of Claims 12 to 23 is applied to the said fibres, for a time that is sufficient to develop the desired coloration, in the presence of an oxidizing agent, the oxidizing agent being applied before, simultaneously with or after the composition.

26. Process for dyeing keratin fibres, and in particular human keratin fibres such as the hair, **characterized in that** the ready-to-use composition according to Claim 24 is applied to the said fibres for a time that is sufficient to develop the desired coloration.

27. Use of a composition comprising, in a medium that is suitable for dyeing, at least one compound of general formula (I) as defined in Claim 1, in which:
the radicals R₁ and R₂ represent, independently of each other, a hydrogen atom, an alkyl group, a monoalkylaminocarbonyl group or a dialkylaminocarbonyl group, or the radicals R₁ and R₂ form, together with the nitrogen on which they are substituent, a saturated 5- or 6-membered ring optionally interrupted with one or more nitrogen atoms or with one or more carbonyl functions, and optionally substituted with one or more alkyl groups,
the radical R represents a linear or branched C₁-C₁₀ alkylene radical, optionally interrupted with one or more atoms chosen from nitrogen and oxygen or with one or more carbonyl functions, and optionally substituted with one or more groups chosen from amino, monoalkylamino, dialkylamino, alkylcarbonyl, amido, monoalkylaminocarbonyl and dialkylaminocarbonyl groups, with the proviso that, when R represents a linear or branched C₁-C₁₀ alkylene radical that is unsubstituted and not interrupted with one or more atoms chosen from nitrogen and oxygen or with one or more carbonyl functions, then the radicals R₁ and R₂ do not represent one of the following couples: two hydrogen atoms, two alkyl groups, a hydrogen atom and an alkyl group, for dyeing fibres, preferably keratin fibres such as the hair.

28. Use of the composition according to Claim 27 in a multi-compartment device, a first compartment containing the cosmetic composition for dyeing keratin fibres, and a second compartment containing an oxidizing agent.

## Patentansprüche

1. Verbindung, **dadurch gekennzeichnet, dass** es sich um ein sekundäres N-alkylaminiertes para-Phenylendiamin der allgemeinen Formel (I) handelt: 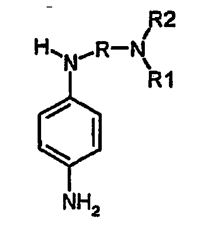 in der Formel:
die Gruppen R₁ und R₂ bedeuten unabhängig voneinander ein Wasserstoffatom, eine Alkylgruppe, eine Monoalkylaminocarbonylgruppe, eine Dialkylaminocarbonylgruppe, oder die Gruppen R₁ und R₂ bilden gemeinsam und mit dem Stickstoffatom, dessen Substituenten sie sind, einen gesättigten 5- oder 6-gliedrigen Ring, der gegebenenfalls durch ein oder mehrere Stickstoffatome oder durch eine oder mehrere Carbonylfunktionen unterbrochen und gegebenenfalls mit einer oder mehreren Alkylgruppen substituiert ist;
die Gruppe R bedeutet eine geradkettige oder verzweigte C₁₋₁₀-Alkylengruppe, die gegebenenfalls durch ein oder mehrere Atome unterbrochen ist, die unter Stickstoff und Sauerstoff ausgewählt sind, und gegebenenfalls mit einer oder mehreren Gruppen substituiert ist, die ausgewählt sind unter Amino, Monoalkylamino, Dialkylamino, Alkylcarbonyl, Amido, Monoalkylaminocarbonyl, Dialkylaminocarbonyl;
mit der Maßgabe, dass die Gruppen R₁ und R₂ keines der folgenden Paare bedeuten: zwei Wasserstoffatome, zwei Alkylgruppen, ein Wasserstoffatom und eine Alkylgruppe, wenn die Gruppe R eine geradkettige oder verzweigte C₁₋₁₀-Alkylengruppe bedeutet, die unsubstituiert vorliegt und nicht durch ein oder mehrere Atome, die unter Stickstoff und Sauerstoff ausgewählt sind, oder nicht durch eine oder mehrere Carbonylgruppen unterbrochen wird;
und mit der Maßgabe, dass es sich bei der Verbindung der Formel (I) weder um das 4-[4-(Piperidinyl)butylamino]aminobenzol noch das N-[3-(4-Methylpiperazin-1-yl)propyl]benzol-1,4-diamin noch das 4-[2-(Piperidinyl)ethylamino]aminobenzol handelt.

2. Verbindung der Formel (I) nach Anspruch 1, bei der R₁ ein Wasserstoffatom bedeutet, wobei R₂ die in Anspruch 1 angegebenen Bedeutungen aufweist.

3. Verbindung der Formel (I) nach Anspruch 1, bei der die Gruppen R₁ et R₂ gemeinsam mit dem Stickstoffatom, dessen Substituenten sie sind, einen gesättigten 5- oder 6-gliedrigen Ring bilden, der gegebenenfalls durch ein oder mehrere Stickstoffatome oder durch eine oder mehrere Carbonylfunktionen unterbrochen und gegebenenfalls mit einer oder mehreren Alkylgruppen substituiert ist.

4. Verbindung nach Anspruch 3, bei der die Gruppen R₁ et R₂ gemeinsam mit dem Stickstoffatom, dessen Substituenten sie sind, einen gesättigten 5- oder 6-gliedrigen Ring bilden, der durch ein oder mehrere Stickstoffatome oder durch eine Carbonylfunktion unterbrochen und gegebenenfalls mit einer oder mehreren Alkylgruppen substituiert ist.

5. Verbindung der Formel (I) nach Anspruch 1, bei der die Gruppe R eine geradkettige C₁₋₇-Alkylengruppe und vorzugsweise C₁₋₃-Alkylengruppe bedeutet.

6. Verbindung der Formel (I) nach Anspruch 5, bei der die Gruppe R unsubstituiert oder durch ein oder mehrere Atome unterbrochen ist, die unter Stickstoff und Sauerstoff ausgewählt sind.

7. Verbindung der Formel (I) nach einem der vorhergehenden Ansprüche, **dadurch gekennzeichnet, dass** sie ausgewählt ist unter: N-[3-(3-Aminopropylamino)-propyl]benzol-1,4-diamin ; N-(2-Pyrrolidin-1-yl-ethyl)-benzol-1,4-diamin ; N-[3-(2-Methylpiperidin-1-yl)-propyl]benzol-1,4-diamin ; N-(3-Pyrrolidin-1-yl-propyl)-benzol-1,4-diamin ; N-[3-(4-Methylpiperazin-1-yl)-propyl]benzol-1,4-diamin ; 1-[3-(4-Aminophenylamino)-propyl]-pyrrolidin-2-on, N-{2-[2-(2-Dimethylaminoethoxy)-ethoxy]-ethyl}benzol-1,4-diamin.

8. Verbindung der Formel (I) nach einem der vorhergehenden Ansprüche, **dadurch gekennzeichnet, dass** sie in der Form eines Salzes vorliegt.

9. Verbindung der Formel (I) nach Anspruch 8, **dadurch gekennzeichnet, dass** sie als Additionssalz mit einer Säure vorliegt, dass unter den Hydrochloriden, Hydrobromiden, Sulfaten, Citraten, Succinaten, Tartraten, Lactaten, Tosylaten, Benzolsulfonaten, Phosphaten und Acetaten ausgewählt ist.

10. Herstellung einer Verbindung nach einem der Ansprüche 1 bis 9, **dadurch gekennzeichnet, dass** die entsprechende nitrierte Verbindung reduziert wird.

11. Verwendung mindestens einer Verbindung der allgemeinen Formel (I) nach einem der Ansprüche 1 bis 9, wobei die Gruppen R₁ und R₂ unabhängig voneinander ein Wasserstoffatom, eine Alkylgruppe, eine Monoalkylaminocarbonylgruppe, eine Dialkylaminocarbonylgruppe bedeuten oder die Gruppen R₁ und R₂ gemeinsam mit dem Stickstoffatom, dessen Substituenten sie sind, einen gesättigten 5- oder 6-gliedrigen Ring bilden, der gegebenenfalls durch ein oder mehrere Stickstoffatome oder durch eine oder mehrere Carbonylfunktionen unterbrochen und gegebenenfalls mit einer oder mehreren Alkylgruppen substituiert ist;
die Gruppe R eine geradkettige oder verzweigte C₁₋₁₀-Alkylengruppe bedeutet, die gegebenenfalls durch ein oder mehrere Atome, die unter Stickstoff und Sauerstoff ausgewählt sind, oder durch eine oder mehrere Carbonylfunktionen unterbrochen ist und gegebenenfalls mit einer oder mehreren Gruppen substituiert ist, die unter Amino, Monoalkylamino, Dialkylamino, Alkylcarbonyl, Amido, Monoalkylaminocarbonyl, Dialkylaminocarbonyl ausgewählt sind;
mit der Maßgabe, dass die Gruppen R₁ und R₂ keines der folgenden Paare bedeuten: zwei Wasserstoffatome, zwei Alkylgruppen, ein Wasserstoffatom und eine Alkylgruppe, wenn die Gruppe R eine geradkettige oder verzweigte C₁₋₁₀-Alkylengruppe bedeutet, die unsubstituiert vorliegt und nicht durch ein oder mehrere Atome, die unter Stickstoff und Sauerstoff ausgewählt sind, oder nicht durch eine oder mehrere Carbonylgruppen unterbrochen wird; zum Färben von Fasern, vorzugsweise Keratinfasern, wie Haaren.

12. Kosmetische Zusammensetzung zum Färben von Keratinfasern und vorzugsweise menschlichen Keratinfasern, wie Haaren, die in einem zum Färben geeigneten Medium mindestens eine Verbindung der allgemeinen Formel (I) nach einem der Ansprüche 1 bis 9 enthält, wobei
die Gruppen R₁ und R₂ unabhängig voneinander ein Wasserstoffatom, eine Alkylgruppe, eine Monoalkylaminocarbonylgruppe, eine Dialkylaminocarbonylgruppe bedeuten oder die Gruppen R₁ und R₂ gemeinsam mit dem Stickstoffatom, dessen Substituenten sie sind, einen gesättigten 5- oder 6-gliedrigen Ring bilden, der gegebenenfalls durch ein oder mehrere Stickstoffatome oder durch eine oder mehrere Carbonylfunktionen unterbrochen und gegebenenfalls mit einer oder mehreren Alkylgruppen substituiert ist;
die Gruppe R eine geradkettige oder verzweigte C₁₋₁₀-Alkylengruppe bedeutet, die gegebenenfalls durch ein oder mehrere Atome, die unter Stickstoff und Sauerstoff ausgewählt sind, oder durch eine oder mehrere Carbonylfunktionen unterbrochen ist und gegebenenfalls mit einer oder mehreren Gruppen substituiert ist, die unter Amino, Monoalkylamino, Dialkylamino, Alkylcarbonyl, Amido, Monoalkylaminocarbonyl, Dialkylaminocarbonyl ausgewählt sind;
mit der Maßgabe, dass die Gruppen R₁ und R₂ keines der folgenden Paare bedeuten: zwei Wasserstoffatome, zwei Alkylgruppen, ein Wasserstoffatom und eine Alkylgruppe, wenn die Gruppe R eine geradkettige oder verzweigte C₁₋₁₀-Alkylengruppe bedeutet, die unsubstituiert vorliegt und nicht durch ein oder mehrere Atome, die unter Stickstoff und Sauerstoff ausgewählt sind, oder nicht durch eine oder mehrere Carbonylfunktionen unterbrochen wird.

13. Zusammensetzung nach Anspruch 12, **dadurch gekennzeichnet, dass** sie mindestens einen kosmetischen Zusatzstoff enthält, der unter den Antioxidationsmitteln, Penetrationsmittel, Maskierungsmitteln, Parfums, Puffern, Dispergiermitteln, grenzflächenaktiven Stoffen, Konditioniermitteln, Filmbildnern, Polymeren, Ceramiden, Konservierungsmitteln, Perlglanzmitteln oder Trübungsmitteln, Vitaminen oder Provitaminen ausgewählt ist.

14. Kosmetische Zusammensetzung zum Färben von Keratinfasern und vorzugsweise menschlichen Keratinfasern, wie Haaren, die in einem zum Färben geeigneten Medium mindestens eine Verbindung der allgemeinen Formel (I) nach einem der Ansprüche 1 bis 9 und mindestens einen kosmetischen Zusatzstoff enthält, der unter den Antioxidationsmitteln, Penetrationsmittel, Maskierungsmitteln, Parfums, Puffern, Dispergiermitteln, grenzflächenaktiven Stoffen, Konditioniermitteln, Filmbildnern, Polymeren, Ceramiden, Konservierungsmitteln, Perlglanzmitteln oder Trübungsmitteln, Vitaminen oder Provitaminen ausgewählt ist.

15. Zusammensetzung nach einem der Ansprüche 12 bis 14, **dadurch gekennzeichnet, dass** die Konzentration der Verbindung der allgemeinen Formel (I) im Bereich von 0,0001 bis 20 % und vorzugsweise 0,005 bis 6 Gew.-%, bezogen auf das Gesamtgewicht der Zusammensetzung, liegt.

16. Zusammensetzung nach einem der Ansprüche 12 bis 15, **dadurch gekennzeichnet, dass** das zum Färben geeignete Medium aus Wasser oder einem Gemisch von Wasser und mindestens einem organischen Lösungsmittel besteht, wie verzweigten oder unverzweigten niederen C₁₋₄-Alkoholen, beispielsweise Ethanol und Isopropanol; Polyolen und Polyolethern, wie 2-Butoxyethanol, Propylenglycol, Propylenglycolmonomethylether, Diethylenglycolmonoethylether und -monomethylether, Glycerin sowie den aromatischen Alkoholen, beispielsweise Benzylalkohol oder Phenoxyethanol, und deren Gemischen.

17. Zusammensetzung nach einem der Ansprüche 12 bis 16, **dadurch gekennzeichnet, dass** der Mengenanteil für jeden kosmetischen Zusatzstoff im Bereich von 0,01 bis 20 Gew.-%, bezogen auf das Gesamtgewicht der Zusammensetzung, liegt.

18. Zusammensetzung nach einem der Ansprüche 12 bis 17, **dadurch gekennzeichnet, dass** sie mindestens ein ergänzendes Farbstoffvorprodukt eines Oxidationsfarbstoffes enthält, das von den Verbindungen der Formel (I) nach einem der Ansprüche 1 bis 9 verschieden ist.

19. Zusammensetzung nach Anspruch 18, **dadurch gekennzeichnet, dass** das ergänzende Farbstoffvorprodukt eines Oxidationsfarbstoffes ein herkömmlicher oxidativer Kuppler ist, der unter den meta-Phenylendiaminen, meta-Aminophenolen, meta-Dihydroxybenzolen, Naphthalinkupplern, heterocyclischen Kupplern sowie ihren Additionssalzen ausgewählt ist.

20. Zusammensetzung nach Anspruch 19, **dadurch gekennzeichnet, dass** die Konzentration des Kupplers im Bereich von 0,0001 bis 20 Gew.-%, bezogen auf das Gesamtgewicht der Zusammensetzung, liegt.

21. Zusammensetzung nach einem der Ansprüche 17 bis 20, **dadurch gekennzeichnet, dass** das ergänzende Farbstoffvorprodukt eines Oxidationsfarbstoffes eine Oxidationsbase ist, die von den Verbindungen der Formel (I) nach einem der Ansprüche 1 bis 9 verschieden ist und unter den para-Phenylendiaminen, Bisphenylalkylendiaminen, para-Aminophenolen, ortho-Aminophenolen und den heterocyclischen Basen und ihren Additionssalzen ausgewählt ist.

22. Zusammensetzung nach Anspruch 21, wobei die Konzentration dieser Oxidationsbase im Bereich von 0,0001 bis 20 Gew.-% des Gesamtgewichts der Farbmittelzusammensetzung liegt.

23. Zusammensetzung nach einem der Ansprüche 12 bis 22, **dadurch gekennzeichnet, dass** sie einen oder mehrere natürliche oder kationische Direktfarbstoffe enthält.

24. Gebrauchsfertige Zusammensetzung, **dadurch gekennzeichnet, dass** es sich um ein Gemisch aus einer Zusammensetzung nach einem der Ansprüche 12 bis 23 und mindestens einem Oxidationsmittel handelt, das unter Wasserstoffperoxid, Harnstoffperoxid, Alkalimetallbromaten, Persalzen, Persäuren und Oxidasen ausgewählt ist.

25. Verfahren zum Färben von Keratinfasern und insbesondere menschlichen Keratinfasern wie Haaren, **dadurch gekennzeichnet, dass** mindestens eine Zusammensetzung nach einem der Ansprüche 12 bis 23 in Gegenwart eines Oxidationsmittels während einer Zeitspanne, die zur Bildung der gewünschten Färbung ausreichend ist, auf die Fasern aufgebracht wird, wobei das Oxidationsmittel vor der, gleichzeitig mit der oder nach der Zusammensetzung aufgetragen wird.

26. Verfahren zum Färben von Keratinfasern und insbesondere menschlichen Keratinfasern wie Haaren, **dadurch gekennzeichnet, dass** die gebrauchsfertige Zusammensetzung nach Anspruch 24 während einer Zeitspanne, die zur Bildung der gewünschten Färbung ausreichend ist, auf die Fasern aufgebracht wird.

27. Verwendung einer Zusammensetzung, die in einem zum Färben geeigneten Medium mindestens eine Verbindung der allgemeinen Formel (I) nach Anspruch 1 enthält, wobei
die Gruppen R₁ und R₂ unabhängig voneinander ein Wasserstoffatom, eine Alkylgruppe, eine Monoalkylaminocarbonylgruppe, eine Dialkylaminocarbonylgruppe bedeuten oder die Gruppen R₁ und R₂ gemeinsam mit dem Stickstoffatom, dessen Substituenten sie sind, einen gesättigten 5- oder 6-gliedrigen Ring bilden, der gegebenenfalls durch ein oder mehrere Stickstoffatome oder durch eine oder mehrere Carbonylfunktionen unterbrochen und gegebenenfalls mit einer oder mehreren Alkylgruppen substituiert ist;
die Gruppe R eine geradkettige oder verzweigte C₁₋₁₀-Alkylengruppe bedeutet, die gegebenenfalls durch ein oder mehrere Atome, die unter Stickstoff und Sauerstoff ausgewählt sind, oder durch eine oder mehrere Carbonylfunktionen unterbrochen ist und gegebenenfalls mit einer oder mehreren Gruppen substituiert ist, die unter Amino, Monoalkylamino, Dialkylamino, Alkylcarbonyl, Amido, Monoalkylaminocarbonyl, Dialkylaminocarbonyl ausgewählt sind;
mit der Maßgabe, dass die Gruppen R₁ und R₂ keines der folgenden Paare bedeuten: zwei Wasserstoffatome, zwei Alkylgruppen, ein Wasserstoffatom und eine Alkylgruppe, wenn die Gruppe R eine geradkettige oder verzweigte C₁₋₁₀-Alkylengruppe bedeutet, die unsubstituiert vorliegt und nicht durch ein oder mehrere Atome, die unter Stickstoff und Sauerstoff ausgewählt sind, oder nicht durch eine oder mehrere Carbonylgruppen unterbrochen wird; zum Färben von Fasern, vorzugsweise Keratinfasern, wie Haaren.

28. Verwendung der Zusammensetzung nach Anspruch 27 in einer Vorrichtung mit mehreren Abteilungen, wobei eine erste Abteilung die kosmetische Zusammensetzung zum Färben von Keratinfasern und eine zweite Abteilung ein Oxidationsmittel enthält.
